# FASCICULE DE BREVET EUROPEEN

(11) **EP 2 872 058 B1**
(45) Date de publication et mention de la délivrance du brevet: **10.04.2019**
(21) Numéro de dépôt: 13735347.0
(22) Date de dépôt: 12.07.2013
(51) Int. Cl.: A61B 17/00, A61B 17/22, A61B 17/29, A61B 17/32, A61B 17/3201

(54) **DISPOSITIF DE DECOUPE POUR CHIRURGIE ENDOVASCULAIRE**
SCHNEIDVORRICHTUNG FÜR ENDOVASKULÄRE CHIRURGIE
CUTTING DEVICE FOR ENDOVASCULAR SURGERY

(30) Priorité: 12.07.2012 FR 1256751
(43) Date de publication de la demande: 20.05.2015
(73) Titulaire: Assistance Publique - Hôpitaux De Paris, 75475 Paris Cedex 10 (FR)
(72) Inventeur: ALSAC, Jean-Marc, F-75017 Paris (FR); DUFETELLE, Edouard, F-75007 Paris (FR)
(74) Mandataire: Vidon Brevets & Stratégie
(86) Numéro de dépôt international: PCT/EP2013/064842
(87) Numéro de publication internationale: WO 2014/009554

(56) Documents cités:
- JP-A- 2002 119 514
- US-A- 5 762 070
- US-A1- 2011 118 769

## Description

### 1. Domaine de l'invention

Le domaine de l'invention est celui des dispositifs médicaux pour la réalisation d'interventions chirurgicales ou médicales. Plus précisément, l'invention concerne un dispositif de découpe permettant la réalisation d'interventions de chirurgie endovasculaire, notamment pour la chirurgie des dissections aigües ou chroniques de l'aorte.

### 2. Art antérieur

La dissection de l'aorte thoraco-abdominale est une pathologie grave, engageant le pronostic vital des patients à court et long terme. Les facteurs de risque de ce type d'accidents cardiovasculaires sont notamment l'hypertension artérielle ou les pathologies congénitales telles que la maladie de Marfan qui affecte les fibres élastiques contenues dans la paroi de l'aorte. L'incidence de cette pathologie est d'environ 1% mais le taux de mortalité des patients atteint 50% dans les 48 premières heures (Midulla et al., Journal of Thoracic and Cardiovascular Surgery (2011) 66-72). Une des explications de la faible incidence de cette pathologie tient au fait que les symptômes de la dissection sont peu spécifiques et sont souvent confondus avec ceux indiquant un infarctus du myocarde. De plus, la douleur thoracique n'est pas nécessairement présente. Le diagnostic de la dissection aortique est alors retardé. Seules les techniques d'imagerie comme l'imagerie par résonnance magnétique, le scanner thoracique ou l'échographie transoesophagienne permettent de confirmer les suspicions du praticien.

D'un point de vue anatomique, la dissection aortique se caractérise par l'irruption de sang à l'intérieur de la paroi aortique. Plus précisément, il se produit d'abord une déchirure au niveau de l'intima, le feuillet interne de la paroi aortique. Le sang affluant du coeur s'engouffre dans la déchirure et, par la pression qu'il exerce sur le tissu, s'immisce entre les feuillets superposés constituant la paroi de l'aorte, également désignée sous le terme de media. Ce faisant, il forme un second chenal dans lequel le sang s'accumule. Parfois, une seconde déchirure, éloignée de la première permet au sang de ressortir et de rejoindre le vrai chenal. Le chenal principal et naturel sera désigné dans la description qui va suivre comme le vrai chenal tandis que le chenal créé par la dissection sera désigné comme le faux chenal.

La classification de DeBakey distingue trois types de dissection en fonction de leur origine anatomique :
- type 1 : la dissection débute sur l'aorte ascendante, se prolonge au niveau de la crosse aortique et se termine sur l'aorte descendante ;
- type 2 : la dissection débute et se termine sur l'aorte ascendante ; et
- type 3 : la dissection débute, se prolonge et se termine sur l'aorte descendante.

La principale complication de la dissection aortique résulte du fait qu'il existe une pression sanguine trop importante dans le faux chenal, comprimant de fait la circulation du sang dans le vrai chenal. Ce gradient de pression entre les deux chenaux est lié à l'existence d'une plus grande résistance opposée au flux sanguin dans le faux chenal. Par conséquent, le sang circule mal au niveau de la portion de l'aorte disséquée et il s'ensuit une mauvaise perfusion des artères irriguant les organes et les membres inférieurs. Si la compression du vrai chenal par le faux chenal n'est pas rapidement levée, les organes dont la perfusion dépend de l'aorte présentent rapidement des dysfonctionnements, parfois irréversibles. Le coeur se fatigue rapidement en essayant d'envoyer dans l'aorte le sang qu'il reçoit, alors que la lumière de l'artère est comprimée.

L'urgence est donc de rétablir l'équilibre des pressions entre les deux chenaux et par là même une circulation sanguine dans les organes vitaux. L'un des traitements d'urgence consiste à fermer la brèche en introduisant une endoprothèse jusqu'au lieu de la dissection, via l'artère fémorale, sous contrôle radioscopique. Les endoprothèses utilisées sont couvertes d'un tissu synthétique afin de bloquer l'accès du faux chenal au sang. Bien qu'efficace, le recours à l'endoprothèse se révèle parfois impossible pour des conditions anatomiques défavorables, ou insuffisant compte tenu de l'existence d'autres porte de réentrée.

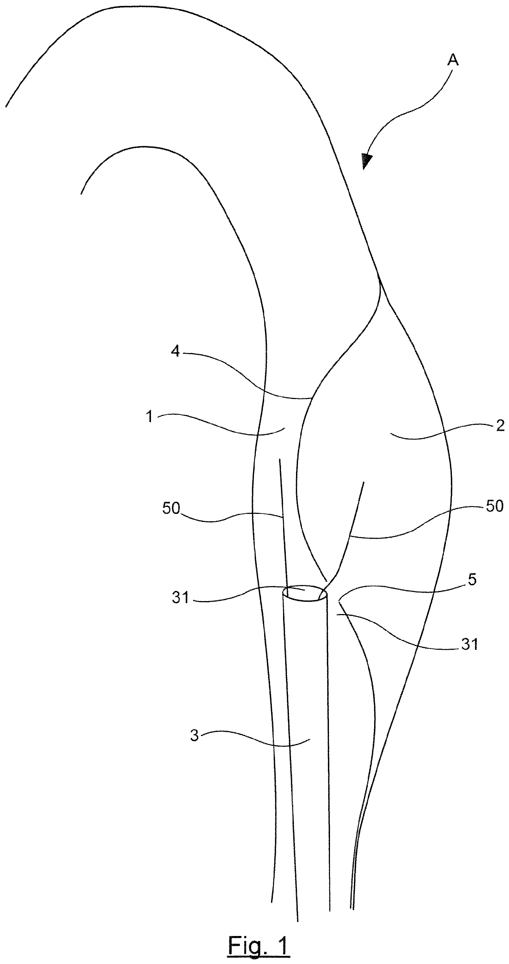

Une autre option consiste à déchirer le flap séparant les chenaux. Cette technique est appelée fenestration et est illustrée à la figure 1. Pour cela, deux tiges de guidage, également appelés « guides », 50 sont introduits dans l'aorte du patient par l'artère fémorale et sont montés sous contrôle radioscopique jusqu'au lieu de la dissection. Un des guides 50 chemine dans le vrai chenal 1 de l'aorte A tandis que le second guide 50 est introduit dans le faux chenal 2 à travers une déchirure 5. Un introducteur 3 d'environ 3 mm de diamètre, comprenant une lumière centrale 31, est ensuite introduit, sur les guides. Une fois que le chirurgien sent que l'introducteur bute contre le flap 4, il exerce une poussée pour le déchirer de manière rétrograde. Le flap ainsi déchiré, la pression s'équilibre entre le vrai et le faux chenal, le vrai chenal 1 est décomprimé et les artères viscérales sont alors rapidement revascularisées. La technique de fenestration se révèle très efficace lorsqu'il s'agit de parer à une situation d'urgence vitale. Toutefois, le geste reste finalement peu maîtrisé par le praticien qui travaille, malgré le suivi radioscopique, « à l'aveugle ».

Cependant, les dangers liés à la dissection aortique ne se limitent pas à la phase aigüe. En effet, dans 60% des cas, la dissection évolue vers un anévrysme disséquant étendu, à savoir la formation d'une poche intrapariétale gorgée de sang, au niveau de la dissection. Ces anévrysmes présentent un risque de rupture important en raison de la forte pression sanguine qui y règne et la dégénérescence de la paroi de l'aorte restante. Lorsque l'anévrysme atteint la taille de 65 mm ou au-delà, le patient présente une indication opératoire à titre préventif, afin d'éviter une rupture brutale qui lui serait fatale. De plus, il est rare que le diagnostic de la dissection se fasse au moment de la phase aigüe. Le flap séparant le vrai chenal du faux chenal est alors complètement rigidifié avec le temps, en raison du développement d'un tissu fibreux cicatriciel.

Le traitement des dissections aortiques en phase chronique implique des contraintes différentes que pour celui des dissections en phase aigüe. Ainsi, le recours aux endoprothèses équipées de branches viscérales est un choix qui ne peut être que rarement mis en pratique dans ce type de dissection chronique anévrysmale. En effet, le déploiement des endoprothèses avec branches viscérales ne peut s'envisager que dans le vrai chenal aortique car la rigidification du flap rend le cathétérisme des artères viscérales souvent impossible. Cette solution est donc complètement exclue.

Une alternative a récemment été proposée : il s'agit de poser, au niveau des anévrysmes disséquant, un stent de type « flow diverter ». Ce dispositif est actuellement utilisé pour soigner des anévrysmes intracrâniens (Pierot, Journal of Neuroradiology (2011), 38, 40-46). De même, ce type d'endoprothèse ne peut être posé que dans le vrai chenal, le faux chenal restant perméable.

La technique de fenestration par un introducteur telle que décrite plus haut est également complètement exclue: si elle donne de bons résultats avec un flap souple pendant les premiers jours suivants la dissection, la déchirure devient impossible voire dangereuse avec un flap rigidifié par le temps. En effet, le chirurgien serait alors obligé de forcer contre le flap qui risquerait de céder brusquement et de fragiliser l'aorte voire d'entrainer sa rupture, ce qui serait fatal pour le patient.

Comme ces traitements sont exclus, il ne reste que la chirurgie à ciel ouvert, c'est à dire que le patient est ouvert au niveau thoraco-abdominal afin que le chirurgien puisse opérer l'aorte directement. Toutefois, les interventions à ciel ouvert sont très lourdes, tant pour le patient que pour l'équipe médicale. Les complications liées à une telle opération sont nombreuses (décès, paraplégie, insuffisance rénale terminale...), la récupération post-opératoire est longue et difficile pour le patient. Elle est également très couteuse pour le système de santé. Ces risques sont d'autant plus élevés que les patients présentent généralement des pathologies sous-jacentes telle que l'hypertension artérielle, et ont une faible condition générale. Il est d'ailleurs fréquent que ces patients soient inéligibles à ce type de chirurgie, les dangers pouvant se révéler supérieurs aux bénéfices.

Il est donc nécessaire de proposer un dispositif permettant d'élargir les indications des actes de chirurgie endovasculaire, limitant le caractère invasif des interventions, et permettant au praticien d'opérer les anévrysmes disséquants aortiques, quelque soit le stade de rigidification du flap.

Le document US 2011/118769 A1 décrit un dispositif de découpe endovasculaire comprenant des "capuchons" placés au niveau de la pointe des lames et dans lesquels des canaux sont ménagés pour y insérer des tiges de guidage.

### 3. Objectifs de l'invention

L'invention a notamment pour objectif de pallier ces inconvénients de l'art antérieur.

Plus précisément, un objectif de l'invention est de fournir, dans au moins un mode de réalisation, un dispositif chirurgical permettant de découper la paroi vasculaire ou « flap » d'une dissection, quelque soit son stade de rigidité.

Un autre objectif de l'invention est de mettre en oeuvre, dans au moins un mode de réalisation, un dispositif chirurgical limitant le caractère invasif de l'acte chirurgical pour le patient.

L'invention a encore pour objectif de proposer, dans au moins un mode de réalisation, un dispositif chirurgical endovasculaire qui permette au praticien d'améliorer la fiabilité et la précision de son intervention.

### 4. Exposé de l'invention

Ces objectifs, ainsi que d'autres qui apparaîtront par la suite, sont atteints à l'aide d'un dispositif de découpe endovasculaire pour la mise en oeuvre d'interventions chirurgicales ou médicales.

Selon l'invention, un tel dispositif comprend deux lames de découpe, des moyens d'actionnement à distance des deux lames de découpe et au moins deux tiges de guidage flexibles, lesdites lames de découpe étant montées de manière coulissante sur lesdites tiges de guidage et lesdits moyens d'actionnement à distance comprenant des moyens de transmission flexibles.

Ainsi, l'invention repose sur une approche tout à fait originale de proposer un dispositif de chirurgie endovasculaire qui permette au praticien de découper, et non d'arracher en un geste approximatif et non maîtrisé, un morceau de paroi vasculaire, communément désigné par le terme flap. Il est important de préciser que ce geste de découpe est un geste décidé et entièrement maîtrisé par le praticien. Il ne s'agit pas de déchirer ou lacérer le flap par un quelconque instrument. Le dispositif selon l'invention permet au praticien de découper parfaitement le flap, en régulant lui-même la force appliquée par les lames sur le flap et la vitesse à laquelle le geste est exécuté. Le geste du praticien est donc beaucoup plus sûr et plus précis ce qui est à la fois beaucoup plus confortable pour le chirurgien, et beaucoup plus sûr pour le patient. En effet, contrairement à l'usage de l'introducteur dans la technique classique de fenestration rétrograde, le dispositif selon l'invention permet d'opérer des dissections aortiques à la fois en phase aigüe, mais aussi en phase chronique lors de laquelle le flap a déjà commencé à se rigidifier.

La flexibilité, ou la souplesse, des moyens de transmission permet d'insérer le dispositif coulissant sur deux tiges de guidage, dans le système artériel vasculaire et d'en suivre les méandres sans risquer de blesser ou de perforer un vaisseau. Bien qu'il soit techniquement difficile de commander à distance le fonctionnement des lames de découpe, la circulation des lames sur les tiges de guidage permet d'une part d'acheminer les lames de découpe de manière fiable et précise jusqu'au contact du flap à découper et d'autre part de stabiliser les lames de découpe afin de pouvoir commander leur fonctionnement à distance.

De plus, grâce au dispositif selon l'invention, les patients peuvent être soignés quelque soit le stade d'évolution de leur pathologie. De plus, un tel dispositif selon l'invention est conçu pour opérer un patient en chirurgie mini-invasive, ce qui permet de limiter le caractère morbide de l'intervention. Il peut également permettre l'emploi d'endoprothèse aortiques pour le traitement endovasculaire d'anévrysmes de l'aorte jusqu'ici accessibles qu'au seul traitement chirurgical à ciel ouvert bien plus délabrant. On entend par chirurgie mini-invasive les procédures chirurgicales permettant au chirurgien d'accéder aux organes internes en pratiquant une incision sur la peau du patient de l'ordre du centimètre, et en utilisant des instruments longs et fins, couplés à un système d'imagerie. Les risques et les complications liés à la chirurgie à ciel ouvert étant évités, un plus grand nombre de patients pourra bénéficier du traitement.

De préférence, le fonctionnement du dispositif de découpe est manuel. Toutefois, le dispositif peut également être mis en oeuvre au moyen de radiofréquence ou d'autres énergies électriques. Selon l'invention, le dispositif doit présenter une étanchéité parfaite tout le long des moyens de transmission. Cette caractéristique est importante car elle permet de préserver l'intrusion de sang dans le mécanisme du dispositif et les éventuels dysfonctionnements qui en résulteraient.

Selon l'invention, les lames de découpe sont traversées longitudinalement par un canal permettant l'insertion des tiges de guidage. L'insertion des tiges de guidage dans un canal creusé à l'intérieur de l'épaisseur des lames permet de guider les lames de manière plus précise et plus stable le long des tiges de guidage. Plus la longueur du canal est longue, plus le coulissement des lames sur les tiges est stable. Le canal dans lequel s'insère une tige de guidage s'étend sur toute la longueur externe de chaque lame de découpe. Ainsi, le geste du praticien est plus maîtrisé et plus sûr. Cette caractéristique contribue donc à améliorer la précision du geste de découpe du flap par le praticien.

En revanche, un canal qui ne s'étend que sur une portion de la longueur des lames de découpe permettra un coulissement plus rapide et plus flexible.

Dans un autre mode de réalisation ne faisant pas partie de l'invention, chacune des lames de découpe comporte au moins un anneau permettant l'insertion des tiges de guidage. Dans ce mode de réalisation, au moins un anneau est fixé sur chacune des lames, les tiges de guidage circulant dans la lumière de l'anneau. Ce mode de réalisation permet un coulissement rapide et flexible du dispositif selon l'invention le long des tiges de guidage.

Dans un mode de réalisation avantageux, les moyens d'actionnement à distance comprennent en outre un élément de commande. Cet élément permet au praticien de manipuler lui-même le dispositif de découpe et d'actionner les lames de découpe au moment opportun, en lui permettant d'ajuster le mouvement des lames et leur vitesse d'actionnement.

De préférence, les moyens de transmission flexible comprennent un câble d'actionnement souple monté coulissant dans un cathéter, ledit câble d'actionnement étant relié à sa partie proximale à un élément de commande et relié à sa partie distale aux deux lames de découpe. Dans ce mode de réalisation, le mouvement relatif du câble d'actionnement est limité par rapport à celui du cathéter. Avantageusement, l'élément de commande comprend une paire de branches, une des branches étant reliée de manière fixe au cathéter et l'autre branche étant articulée avec l'élément d'actionnement, les branches étant reliées et articulées entre elles. Cette caractéristique permet au praticien, par la simple manipulation de la paire de branche de faire coulisser le câble d'actionnement à l'intérieur du cathéter, ce mouvement relatif du câble par rapport au cathéter commandant l'ouverture et la fermeture des lames de découpe. De manière encore plus préférée, l'élément de commande comprend une paire de branches choisie parmi les branches à pinces ou les branches à anneaux.

De préférence, les lames de découpe sont articulées sur le câble d'actionnement.

Avantageusement, les lames de découpe présente un angle d'ouverture α compris entre 0 et 60°. L'angle α est nul lorsque les lames sont en position fermée et que le dispositif chemine dans les vaisseaux jusqu'au lieu de l'opération et l'angle α est non nul en position ouverte. Cette caractéristique permet de manipuler le dispositif selon l'invention, et en particulier d'actionner les lames de découpe au sein du vaisseau, sans blesser les parois vasculaires internes et créer de lésions inutiles. Ce paramètre est d'autant plus important que les parois internes sont déjà fragilisées.

Dans un mode de réalisation préféré, les deux lames de découpe tournent sur leur axe longitudinal selon un angle β compris entre 0 et 360°. Cette caractéristique offre un degré supplémentaire de manipulation des deux lames de découpe, permettant d'accéder plus facilement aux lésions.

De préférence, les tiges de guidages sont en polytétrafluoroéthylène (PTFE) ou en nitinol. Ce matériau présente l'avantage d'être biocompatible : son introduction dans le corps humain ne présente aucune innocuité pour le corps humain. De plus, ce matériau est souple et résistant, ce qui permet de le manipuler à travers les méandres du système vasculaire aisément sans risque de rupture. Enfin, le PTFE permet de fabriquer des tiges de guidage très lisses ce qui facilite grandement le glissement des lames de découpe. L'absence d'aspérité sur les tiges de guidage évite aux lames d'accrocher le long des tiges, limitant le frottement entre le matériau des tiges de guidage et le matériau constituant les lames de découpe. Ainsi, le dispositif de découpe est acheminé au lieu de l'intervention par un geste sûr et parfaitement maîtrisé du praticien. Dans un mode de réalisation davantage préféré, les matériaux constituant le dispositif sont choisis parmi l'acier et le nitinol. Ces matériaux présentent l'avantage d'être biocompatible, résistants à la corrosion et à la stérilisation.

Un autre aspect, ne faisant pas partie de l'invention, comprend également un procédé de fenestration des dissections aortiques, dont le but est de découper la paroi vasculaire séparant le vrai chenal du faux chenal, et comprend les étapes suivantes :
- introduction de deux tiges de guidage dans par une voie vasculaire du patient ;
- positionnement d'une des tiges de guidage dans le vrai chenal et positionnement de l'autre tige de guidage dans le faux chenal ;
- insertion de chacune des tiges de guidage dans chacune des deux lames de découpes en dehors du patient ;
- introduction du dispositif de découpe endovasculaire selon l'invention par la voie vasculaire du patient ;
- acheminement du dispositif selon l'invention à l'intérieur des voies vasculaires jusqu'au contact de la paroi vasculaire séparant le vrai chenal du faux chenal ;
- actionnement des lames de découpe grâce aux moyens d'actionnement à distance, et découpe active de la portion de paroi vasculaire souhaitée ;
- retrait du dispositif de découpe et des tiges de guidage de l'organisme du patient.

Ainsi, le procédé de fenestration selon l'invention permet d'opérer les dissections aortiques, qu'elles soient dans leur phase aigüe ou leur phase chronique.

### 5. Liste des figures

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :
- la figure 1 illustre la technique classique de fenestration sur une dissection à la phase aigue.
- la figure 2 représente une vue d'ensemble d'un mode de réalisation du dispositif selon l'invention, en position ouverte.
- la figure 3 représente une vue des lames de découpe selon la figure 2 en position ouverte.
- la figure 4 représente une vue des lames de découpe selon la figure 2 en position fermée.
- la figure 5 présente une vue de la poignée de commande d'un dispositif selon l'invention.
- la figure 6 illustre une variante d'un dispositif selon l'invention.
- La figure 7 présente une vue d'une lame de découpe.

### 6. Description d'un mode de réalisation de l'invention

Le principe général de l'invention repose sur la mise en oeuvre d'un dispositif de découpe endovasculaire comprenant deux lames de découpe, permettant de découper n'importe quelle paroi vasculaire quelque soit son stade de rigidité, montées de manière coulissante sur deux tiges de guidages qui font office de rails pour acheminer les lames de découpe au contact de la paroi à découper. Les moyens de commande à distance autorise le recours à la chirurgie mini-invasive tandis que les moyens de transmission souple et flexible permettent de diriger le dispositif sans craindre de blesser ou perforer la paroi vasculaire.

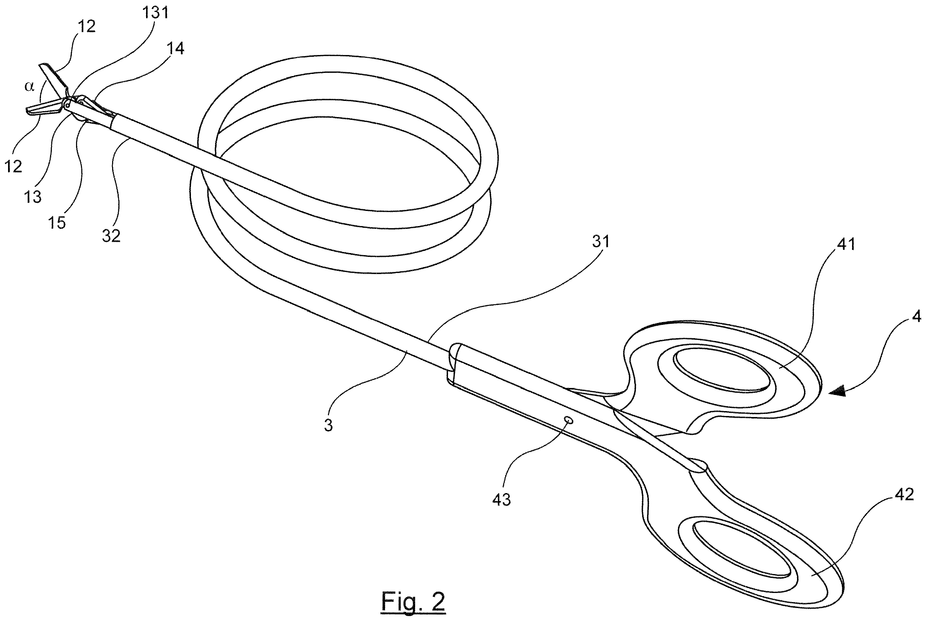

On présente, en relation avec la figure 2, une vue générale d'un mode de réalisation du dispositif selon l'invention, adapté pour opérer une dissection aortique dans sa phase aigüe aussi bien qu'au stade de l'anévrysme disséquant.

Tel que cela est représenté, le dispositif selon l'invention comprend un élément de commande 4 comprenant une paire de branches à anneaux 41, 42, les branches étant croisées et articulées entre elles grâce à un axe 43. L'élément de commande 4 est relié à un cathéter 3 au niveau de la partie proximale du cathéter 31.

Un tel cathéter aura préférentiellement un diamètre compris entre 5 et 7 mm. A l'intérieur du cathéter est logé un câble d'actionnement non représenté sur la figure pour des raisons de clarté. Le diamètre du câble d'actionnement sera préférentiellement compris entre 4 et 6 mm pour pouvoir coulisser dans le cathéter 3. L'ensemble cathéter et câble d'actionnement présentent une longueur comprise entre 40 mm et 100 mm. La longueur du cathéter et du câble d'actionnement dépend de la localisation anatomique de la lésion à opérer. Ce paramètre sera aisément déterminé par l'homme du métier, en faisant appel à ses connaissances générales. Le cathéter ainsi que le câble d'actionnement qu'il contient sont tous deux souples et flexibles. Cette caractéristique permet notamment de naviguer aisément dans le réseau vasculaire sans risquer de le léser.

Le cathéter 3 est lié à sa partie distale 32 avec les deux lames de découpe 11, 12 par l'intermédiaire d'un élément de fixation 13. Comme représenté à la figure 2, les lames de découpe 11, 12 sont écartées l'une de l'autre et forme un angle α. Lorsque les lames de découpe sont écartées et que l'angle α est non nul, elles sont en position ouverte. Lorsque l'angle α est nul, les lames de découpe sont en position fermée. Les lames sont d'ailleurs introduites dans l'organisme en position fermée. De préférence, les lames de découpe présentent chacune une extrémité proximale arrondie, afin de ne pas blesser les parois vasculaires lors de leur fonctionnement ou de leur cheminement. Les lames de découpe sont croisées et articulées entre elles par un axe 131, ce même axe 131 servant à les articuler avec la pièce de fixation 13. Chaque lame de découpe 11, 12 est articulée au niveau de sa partie distale avec une biellette 15, 14. Les tiges de guidage 50 ne sont pas représentées sur cette vue. Les lames de découpe, les biellettes et la pièce de fixation sont réalisées en un matériau métallique biocompatible tel que l'acier ou le nitinol. Il est à noter que le dispositif est représenté, sur cette vue, enroulé sur lui-même pour illustrer la souplesse du câble d'actionnement et du cathéter, et non pour signifier que le dispositif

### a) Exemple d'un premier mode de réalisation

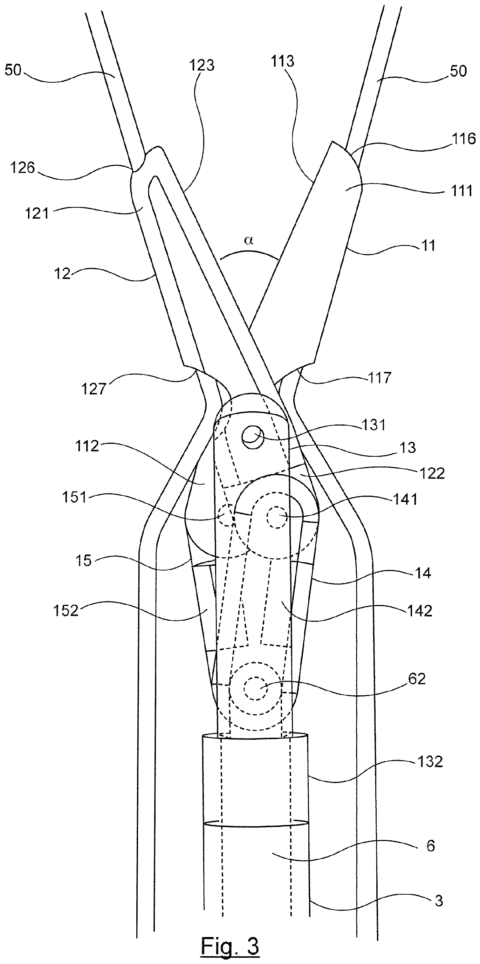

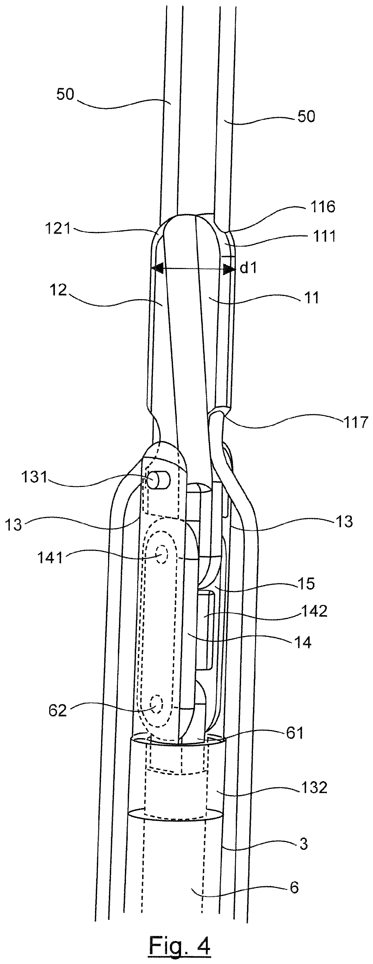

Les figures 3 et 4 représentent une vue détaillée des lames de découpe d'un premier mode de réalisation selon l'invention, respectivement en position ouverte ou fermée. Les parties cachées sont représentées en pointillés. Chacune des lames de découpe 11, 12 possède une extrémité distale 111, 121 arrondie ainsi qu'une extrémité proximale 112, 122. Cette forme arrondie permet d'introduire les lames de découpe en position fermée dans l'organisme sans créer de lésions au niveau de la paroi interne des vaisseaux dans lequel le dispositif circule. Elle permet également d'actionner les lames de découpe en toute sécurité au sein d'un vaisseau. Cette caractéristique est particulièrement importante lorsqu'il s'agit d'opérer un patient présentant déjà des lésions intravasculaires. Chaque lame présente un bord tranchant 113, 123 permettant de découper la paroi vasculaire, y compris lorsqu'un tissu fibreux cicatriciel a commencé à se développer. Les lames sont articulées entre elles par un axe 131, qui les maintient également à la pièce de fixation 13. Chaque lame 11, 12 est articulée au niveau de sa partie proximale à une biellette 14, 15, la lame 11 étant reliée à la biellette 15 par un axe 151 et la lame 12 étant reliée à la lame 14 par un axe 141. Afin de bloquer leur mouvement l'une contre l'autre, et de maintenir les lames 11, 12 alignées l'une contre l'autre dans la position fermée, chaque biellette 14, 15 comprend une butée 142, 152. Les biellettes 14, 15 sont articulées entre elles par un axe 62. Cet axe 62 leur permet également de coopérer avec le câble d'actionnement 6 au niveau de sa partie distale 61. La pièce de fixation 13 présente dans sa partie proximale 132 une bague lui permettant de s'emmancher à force avec le cathéter 3.

Selon l'invention les lames sont montées coulissantes le long de deux tiges de guidage 50. Ces tiges de guidage 50, souples, sont réalisées en nitinol ou en PTFE et présentent une section circulaire de diamètre de 0.035 inch, soit un diamètre d'environ 1 mm. On peut citer, à titre d'exemple de tiges de guidage convenant à la mise en oeuvre de l'invention, les références commerciales Lunderquist-Ring Torque Wire Guides, Cook Inc. ou Radifocus® Guide Wire, Terumo. Les tiges de guidages sont introduites, à l'extérieur du patient, dans les orifices 117, 127 des canaux traversant chacune des lames de découpe 11, 12 pour en ressortir par les orifices 116, 126. Selon l'invention les canaux s'étendent sensiblement selon l'axe longitudinal de chaque lame 11, 12 sur toute la longueur des lames. Il est à noter que les lames de découpe présentent un angle d'ouverture a, mesuré entre les axes longitudinaux de chaque lame 11, 12, ne pouvant excéder 60°. Cet angle d'ouverture est limité afin de ne pas écarter de manière trop importante les lames de découpe l'une de l'autre et de risquer de blesser la paroi interne des vaisseaux. Les lames de découpe peuvent également être mobiles selon un angle β de 360° autour de leur axe longitudinal (non représenté sur les figures).

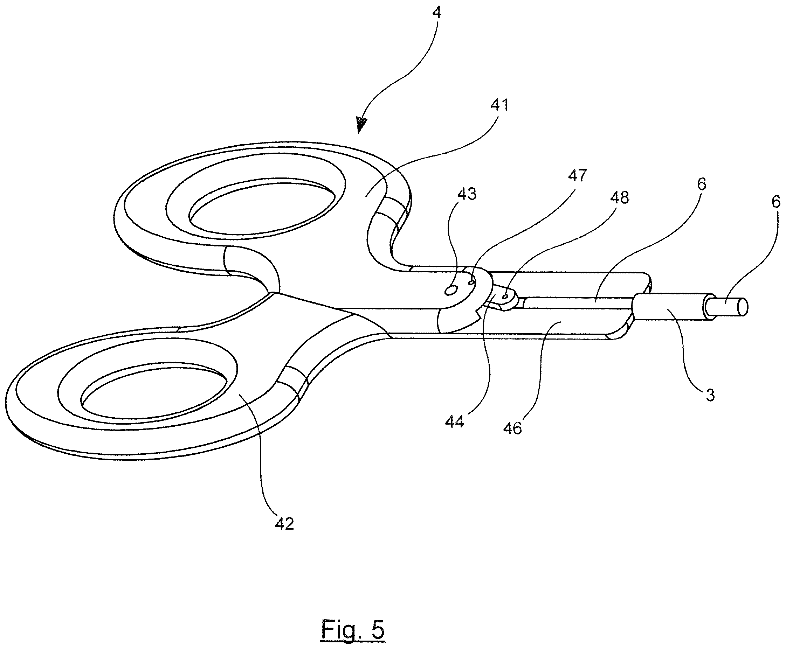

La figure 5 représente une vue schématique de l'élément de commande 4 d'un dispositif selon l'invention. Cet élément de commande 4 comprend une paire de branches à anneaux 41, 42. Les anneaux permettent la préhension du dispositif par le chirurgien. Les branches 41, 42 sont articulées entre elles par un axe 43. Cet axe permet également de relier chacune des branches à une partie plane 46. La branche 42 est donc reliée à la partie plane par l'intermédiaire de l'axe 43, cette partie plane étant relié au cathéter 3 par collage, thermoformage ou soudure. La branche 42 est quand à elle articulée au câble d'actionnement 6 par l'intermédiaire d'une biellette 44. Plus précisément, la branche 42 est articulée à la biellette 44 via un axe 47 tandis que la biellette 44 est également articulée avec le câble d'actionnement 6 par un axe 48. Chaque branche à anneaux comprend en outre une butée, tel qu'il est classiquement mis en oeuvre dans les paires de ciseaux.

Ainsi, lorsque le chirurgien écarte les anneaux de chacune des branches 41, 42 l'un de l'autre, le câble 6 est tiré vers le chirurgien, à l'intérieur du cathéter 3 qui reste fixe. Le mouvement relatif du câble dans le cathéter permet de commander à distance les lames de découpe. Plus exactement, la pièce 61 tire les biellettes 14,15 qui entre en contact avec la bague 132 de la pièce de fixation. La bague bloque la progression en arrière des biellettes ce qui provoquent l'écartement des parties distales 142, 152 de chacune des biellettes 14, 15. Ce mouvement provoque également l'écartement des lames de découpe 11, 12 qui se retrouvent en position ouverte comme représenté à la figure 3. De même, le rapprochement des branches à anneaux provoque l'alignement des biellettes 14, 15 ainsi que celui des lames de découpe 11, 12 avec l'axe longitudinal du câble 6 et du cathéter 3, comme représenté à la figure 4. Ainsi, le chirurgien peut, par un mouvement simple d'ouverture de ciseaux, découper simplement, rapidement et efficacement le flap séparant le vrai chenal du faux chenal. Le bord 123, 113 de chaque lame de découpe 11, 12 est suffisamment tranchant pour opérer les morceaux de paroi vasculaire rigidifiée par le développement d'un tissu cicatriciel. De plus, les lames de découpe 11, 12 sont stabilisées lors de leur fonctionnement et de leur actionnement grâce à la présence des tiges de guidage.

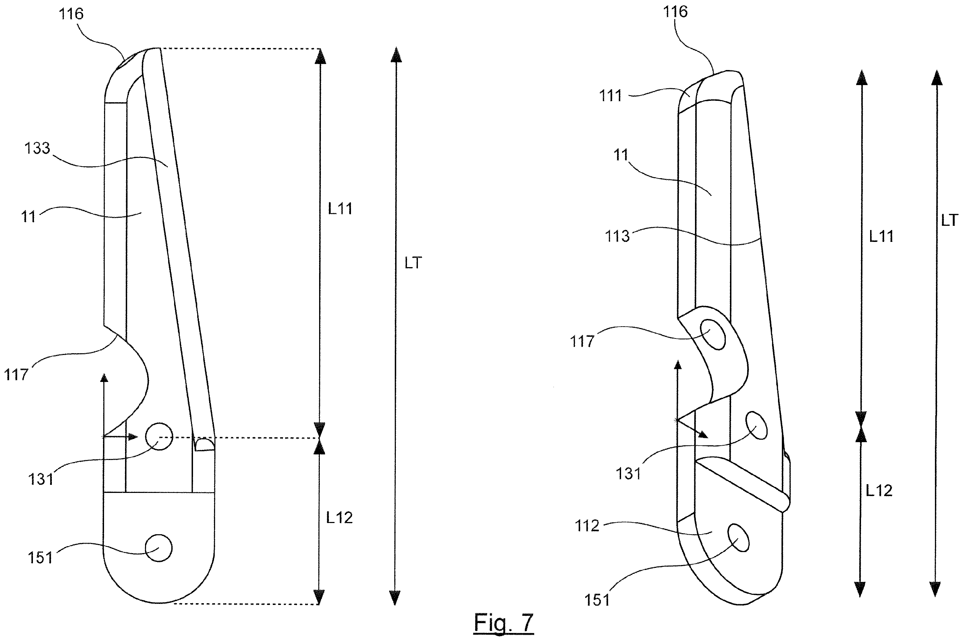

Les dimensions des différents éléments constituant le dispositif selon l'invention dépendent directement de leur destination anatomique et des caractéristiques des vaisseaux dans lesquels ils seront introduits. Celle des lames de découpe sont indiquées à la figure 7. Par exemple, pour découper le flap d'une dissection aortique, les dimensions du dispositif pourront être les suivantes :
- diamètre du cathéter : 5 à 7 mm
- diamètre des tiges de guidage : 1 mm
- diamètre du câble de transmission : 5 mm
- diamètre dl des lames de découpe : 5 mm
- longueur L1 des lames de découpe : 3 cm
- longueur L11 des lames ; 1 cm
- longueur L12 des lames : 2 cm
- longueur des biellettes L2 14,15 : 2 cm
- longueur L3 de la pièce de fixation 13 :
- longueur totale L4 : au maximum 5 cm
- longueur du cathéter 3 : 40 à 100 cm

### b) Exemple d'un second mode de réalisation

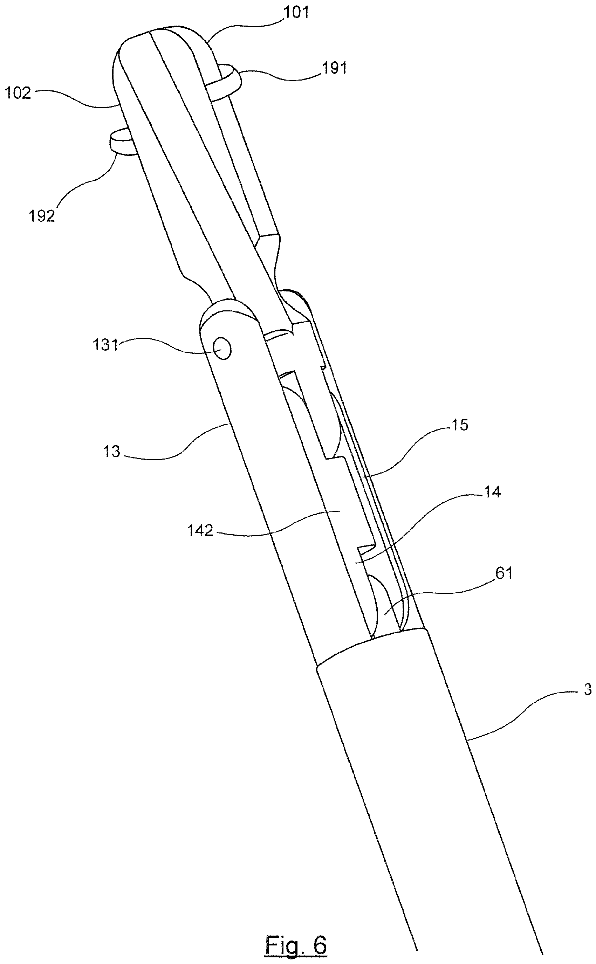

Selon ce second mode de réalisation, ne faisant partie de l'invention, représenté à la figure 6, les lames de découpe 101, 102 sont pleines et coulissent le long des tiges de guidage 500 grâce à des anneaux 191, 192. L'épaisseur de l'anneau est d'environ 2 mm, leur diamètre externe est approximativement de 2 mm.

Le fonctionnement et l'articulation des lames de découpe avec les biellettes 14, 15, la pièce de fixation 13, le câble 6, le cathéter 3 et l'élément de commande 4 sont identiques en tout point avec l'exemple décrit au paragraphe 5a). Les dimensions des pièces sont identiques au premier mode de réalisation.

### 7. Conclusion

Le dispositif selon l'invention permet donc d'opérer de manière peu invasive un patient souffrant d'une dissection aortique ou d'un anévrysme disséquant aortique. Ce dispositif permet donc d'appliquer la technique de fenestration, jusque là réservée à l'ouverture des flaps souples des dissection en phase aigüe, au traitement des anévrysmes survenant sur dissection chronique. Cette progression est permise par la coopération de deux lames de découpe active permettant de découper les flaps, quelque soit leur stade de rigidité, commandé à distance par un élément de commande manipulé par le chirurgien relié aux lames de découpe par l'intermédiaire d'un câble d'actionnement. La souplesse du cathéter et du câble d'actionnement sont des caractéristiques primordiales du dispositif car elle permet d'introduire et de diriger le dispositif selon l'invention à travers les méandres du système vasculaire sans risque de blesser le patient. Enfin, le coulissement des lames de découpe le long de tiges de guidage permet à la fois d'amener les lames au contact du flap à découper à distance, en le positionnant le mieux possible auprès du flap, et de stabiliser les lames de découpe lorsque le chirurgien actionne l'élément de commande.

Ainsi, le chirurgien maitrise mieux son geste, la découpe est plus précise. Les risques liés à la déchirure du flap rigidifié sont écartés. La technique de fenestration peut désormais s'appliquer à tous les patients, quelque soit le stade d'évolution de leur pathologie. Les patients, inéligibles à l'opération à ciel ouvert de par leur mauvaise condition physique, peuvent désormais être soignés.

## Revendications

1. Dispositif de découpe endovasculaire pour la mise en oeuvre d'interventions chirurgicales ou médicales comprenant deux lames de découpe (11, 12, 101, 102), des moyens d'actionnement à distance des deux lames de découpe (11,12, 101, 102) et au moins deux tiges de guidage flexibles (50), lesdites lames de découpe (11,12, 101, 102) étant montées de manière coulissante sur lesdites tiges de guidage (50) et lesdits moyens d'actionnement à distance comprenant des moyens de transmission flexibles (3, 6),
**caractérisé en ce que** lesdites lames de découpe (11, 12, 101, 102) sont traversées sur toute leur longueur externe par un canal creusé à l'intérieur de l'épaisseur des lames permettant l'insertion des tiges de guidage flexibles (50).

2. Dispositif selon la revendication 1 dans lequel les moyens d'actionnement à distance comprennent en outre un élément de commande (4).

3. Dispositif selon la revendication 1 ou 2 dans lequel les moyens de transmission flexibles comprennent un câble d'actionnement (6) souple monté coulissant dans un cathéter (3), ledit câble d'actionnement (6) étant relié à sa partie proximale à l'élément de commande (4) et relié à sa partie distale (61) aux deux lames de découpe (11,12, 101, 102).

4. Dispositif selon la revendication 3 dans lequel l'élément de commande (4) comprend une paire de branches (41, 42), une des branches (42) étant reliée de manière fixe au cathéter (3) et l'autre branche (41) étant articulée avec le câble d'actionnement (6), les branches étant reliées et articulées entre elles.

5. Dispositif selon l'une des revendications 3 ou 4 dans lequel les lames de découpe (11,12, 101, 102) sont articulées sur le câble d'actionnement (6).

6. Dispositif selon l'une des revendications précédentes dans lequel les lames de découpe (11, 12, 101, 102) présentent un angle d'ouverture α compris entre 0 et 60°.

7. Dispositif selon l'une des revendications précédente selon lequel les deux lames de découpe (11, 12, 101, 102) tournent sur leur axe longitudinal selon un angle β compris entre 0 et 360°.

## Patentansprüche

1. Endovaskuläre Schneidvorrichtung für den Einsatz von chirurgischen oder medizinischen Eingriffen, umfassend zwei Schneidblätter (11, 12, 101, 102), Mittel zur Fernbetätigung der zwei Schneidblätter (11, 12, 101, 102) und mindestens zwei flexible Führungsstangen (50), wobei die Schneidblätter (11, 12, 101, 102) gleitend auf den Führungsstangen (50) montiert sind, und die Fernbetätigungsmittel flexible Übertragungsmittel (3, 6) umfassen, **dadurch gekennzeichnet, dass** die Schneidblätter (11, 12, 101, 102) auf ihrer gesamten Außenlänge von einem im Inneren der Dicke der Schneidblätter ausgehöhlten Kanal durchzogen sind, der das Einsetzen der flexiblen Führungsstangen (50) ermöglicht.

2. Vorrichtung nach Anspruch 1, wobei die Fernbetätigungsmittel ferner ein Steuerelement (4) umfassen.

3. Vorrichtung nach Anspruch 1 oder 2, wobei die flexiblen Übertragungsmittel ein biegsames Betätigungskabel (6) umfassen, das in einem Katheter (3) gleitet, wobei das Betätigungskabel (6) in seinem proximalen Teil mit dem Steuerelement (4) und in seinem distalen Teil (61) mit zwei Schneidblättern (11, 12, 101, 102) verbunden ist.

4. Vorrichtung nach Anspruch 3, wobei das Steuerelement (4) ein Paar Schenkel (41, 42) umfasst, wobei einer der Schenkel (42) fest mit dem Katheter (3) verbunden ist, und der andere Schenkel (41) an dem Betätigungskabel (6) angelenkt ist, wobei die Schenkel miteinander verbunden und aneinander angelenkt sind.

5. Vorrichtung nach einem der Ansprüche 3 oder 4, bei der die Schneidblätter (11, 12, 101, 102) an dem Betätigungskabel (6) angelenkt sind.

6. Vorrichtung nach einem der vorhergehenden Ansprüche, bei der die Schneidblätter (11, 12, 101, 102) einen Öffnungswinkel α zwischen 0 und 60° aufweisen.

7. Vorrichtung nach einem der vorhergehenden Ansprüche, bei der sich die zwei Schneidblätter (11, 12, 101, 102) auf ihrer Längsachse um einen Winkel β zwischen 0 und 360° drehen.

## Claims

1. Cutting device for endovascular surgical or medical operations comprising two cutting blades (11, 12, 101, 102), means of remote actuation of two cutting blades (11,12, 101, 102) and at least two flexible guide rods (50), said cutting blades (11,12, 101, 102) being installed free to slide on said guide rods (50) and said remote actuation means including flexible transmission means (3, 6), a channel passing longitudinally through the entire external length of said cutting blades (11, 12, 101, 102) for insertion of the flexible guide rods (50).

2. Device according to claim 1 in which the remote actuation means also include a control element (4).

3. Device according to claim 1 or 2, in which the flexible transmission means include a flexible actuation cable (6) mounted free to slide in a catheter (3), said actuation cable (6) being connected at its proximal part to the control element (4) and at its distal part (61) to the two cutting blades (11,12, 101, 102).

4. Device according to claim 3 in which the control element (4) includes a pair of branches (41, 42), one of the branches (42) being rigidly fixed to the catheter (3) and the other branch (41) being hinged to the actuation element (6), the branches being connected and hinged to each other.

5. Device according to one of claims 3 or 4 in which the cutting blades (11,12, 101, 102) are hinged on the actuation cable (6).

6. Device according to one of the previous claims in which the cutting blades (11, 12, 101, 102) have an opening angle α between 0 and 60°.

7. Device according to one of the previous claims in which the two cutting blades (11, 12, 101, 102) rotate about their longitudinal axis at an angle β between 0 and 360°.
